(19) Europäisches Patentamt / European Patent Office / Office européen des brevets

(11) **EP 4 201 919 A1**

(12) **EUROPEAN PATENT APPLICATION**
published in accordance with Art. 153(4) EPC


(43) Date of publication:
**28.06.2023 Bulletin 2023/26**

(21) Application number: **21858385.4**

(22) Date of filing: **19.08.2021**

(51) International Patent Classification (IPC):
***C07C 67/54** (2006.01)* ***C07C 69/653** (2006.01)*

(52) Cooperative Patent Classification (CPC):
**C07C 67/54; C07C 69/653**

(86) International application number:
**PCT/JP2021/030466**

(87) International publication number:
**WO 2022/039241 (24.02.2022 Gazette 2022/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.08.2020 JP 2020138908**



(71) Applicant: **Daikin Industries, Ltd.**
**Osaka-shi, Osaka 530-0001 (JP)**

(72) Inventors:
- **HOSOKAWA, Moe**
  **Osaka-shi, Osaka 530-0001 (JP)**
- **GOTOU, Akihiro**
  **Osaka-shi, Osaka 530-0001 (JP)**
- **YOSHIYAMA, Asako**
  **Osaka-shi, Osaka 530-0001 (JP)**
- **MATSUURA, Makoto**
  **Osaka-shi, Osaka 530-0001 (JP)**
- **KISHIKAWA, Yosuke**
  **Osaka-shi, Osaka 530-0001 (JP)**

(74) Representative: **Hoffmann Eitle**
**Patent- und Rechtsanwälte PartmbB**
**Arabellastraße 30**
**81925 München (DE)**


(54) **METHOD FOR PURIFYING ACRYLIC ACID ESTER COMPOUND**


(57) The present disclosure addresses the problem of providing a method for purifying an acrylic acid ester compound. This problem can be solved by a method for purifying a compound represented by formula (1)

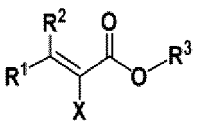 (1)

(wherein $R^1$ and $R^2$ are the same as or different and each represent an alkyl group, a fluoroalkyl group, an aryl group optionally having one or more substituent groups, a halogen atom, or a hydrogen atom, $R^3$ is an alkyl group, a fluoroalkyl group, or an aryl group optionally having one or more substituent groups, and X is a fluoroalkyl group or a halogen atom), the method including step A of distilling composition A containing (a) the compound represented by formula (1), (b) an alcohol, and (c) a salt so as to obtain a concentrate containing the compound represented by formula (1).


EP 4 201 919 A1

## Description

Technical Field

The present disclosure relates to a method for purifying an acrylic ester compound etc.

Background Art

Patent Literature (PTL) 1 discloses a method for separating methanol from a mixture.

Citation List

Patent Literature

PTL 1: JPS62-132925A

Summary of Invention

Technical Problem

An object of the present disclosure is to provide a method for purifying an acrylic ester compound etc.

Solution to Problem

Item 1.

A method for purifying a compound represented by formula (1):

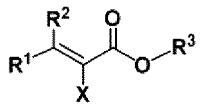 (1)

(wherein $R^1$ and $R^2$ are the same or different and represent an alkyl group, a fluoroalkyl group, an aryl group optionally having one or more substituent groups, a halogen atom, or a hydrogen atom,
$R^3$ is an alkyl group, a fluoroalkyl group, or an aryl group optionally having one or more substituent groups, and
X is a fluoroalkyl group or a halogen atom),
the method comprising step A of distilling composition A containing

(a) the compound represented by formula (1),
(b) an alcohol, and
(c) a salt

to obtain a concentrate containing the compound of formula (1).

Item 2.

The method according to Item 1, wherein composition A further contains water.

Item 3.

The method according to Item 1 or 2, wherein composition A is homogeneous.

Item 4.

The method according to any one of Items 1 to 3, wherein the salt (c) is at least one member selected from the group consisting of inorganic salts and organic salts.

Item 5.

The method according to any one of Items 1 to 4, wherein the quantitative amount ratio of the alcohol (b) to the salt (c) (alcohol (b)/salt (c)) is 0.01 mol/mol or more.

Item 6.

The method according to any one of Items 1 to 5, wherein the salt (c) is solvated in the alcohol (b) that is used in a quantitative ratio of 0.5 mol/mol or more.

Item 7.

The method according to any one of claims 1 to 6, wherein the salt (c) comprises at least one cation selected from the group consisting of

metal cations,
$NR_4^+$ wherein R in each occurrence is the same or different and represents H or an organic group, or any two of the Rs may be linked together to form an optionally substituted ring,
optionally substituted imidazolium,
optionally substituted pyridinium, and
optionally substituted phosphonium.

Item 8.

The method according to any one of Items 1 to 7, wherein the salt (c) comprises at least one cation selected from the group consisting of monovalent metal cations, divalent metal cations, and trivalent metal cations.

Item 9.

The method according to any one of Items 1 to 8, wherein the salt (c) comprises at least one anion selected from the group consisting of carbonate ions, hydrogen carbonate ions, carboxylate ions, sulfate ions, hydroxide ions, halogen ions, and nitrate ions.

Item 10.

The method according to any one of Items 1 to 9, wherein the salt (c) is at least one member selected from the group consisting of LiBr, LiI, $MgBr_2$, $MgI_2$, $ZnCl_2$, $ZnBr_2$, $AlCl_3$, $CaCl_2$, $MgCl_2$, LiCl, NaCl, and NaI.

Item 11.

The method according to any one of Items 1 to 9, wherein the salt (c) is at least one member selected from the group consisting of calcium stearate, calcium tartrate, calcium propionate, and calcium salts of amino acids.

Item 12.

The method according to any one of Items 1 to 11, wherein composition A contains the salt (c) at a concentration of 50% or more, relative to its saturation concentration.

Item 13.

The method according to any one of Items 1 to 12, wherein the concentration of the salt (c) in composition A is 10 wt.% or more.

Item 14.

The method according to any one of Items 1 to 13, wherein the alcohol (b) is a linear or branched alcohol having 1 to 5 carbon atoms.

Item 15.

The method according to any one of Items 1 to 14, wherein the alcohol (b) is represented by formula:

$R^4$-OH (2)

(wherein $R^4$ is an alkyl group or a fluoroalkyl group).

Item 16.

The method according to any one of Items 1 to 15, wherein composition A is directly distilled.

Item 17.

The method according to any one of Items 1 to 16, wherein the distillation is performed at a pressure of 0 to 100 kPa.

Item 18.

The method according to any one of Items 1 to 17, wherein the distillation is performed at a temperature of 10 to 100°C.

Item 19.

The method according to any one of Items 1 to 18, wherein $R^1$ is a hydrogen atom, an alkyl group, or a fluoroalkyl group.

Item 20.

The method according to any one of Items 1 to 19, wherein $R^2$ is a hydrogen atom, an alkyl group, or a fluoroalkyl group.

Item 21.

The method according to any one of Items 1 to 20, wherein $R^3$ is an alkyl group.

Item 22.

The method according to any one of Items 1 to 21, wherein $R^3$ is a $C_{1-4}$ alkyl group.

Item 23.

The method according to any one of Items 1 to 22, wherein X is a fluorine atom or a chlorine atom.

Item 24.

The method according to any one of Items 1 to 23, further comprising step B of washing the concentrate obtained in step A with water or water containing an alkali metal compound or an alkaline earth metal compound, or containing both.

Item 25.

The method according to any one of Items 1 to 23, further comprising step B of washing the concentrate obtained in step A with water or water containing at least one member selected from the group consisting of lithium chloride, lithium bromide, magnesium bromide, magnesium iodide, magnesium sulfate, lithium iodide, sodium iodide, magnesium chloride, and calcium chloride.

Item 26.

The method according to any one of Items 1 to 23, further comprising step B of washing the concentrate obtained in step A with water containing calcium chloride.

Item 27.

The method according to any one of Items 1 to 26, further comprising step C of distilling the residue after obtaining the concentrate in step A so as to recover the alcohol and the salt.

Item 28.

A composition comprising

(a) a compound represented by formula (1):

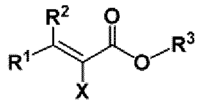
(1)

wherein

$R^1$ and $R^2$ are the same or different and represent an alkyl group, a fluoroalkyl group, an aryl group optionally having one or more substituent groups, a halogen atom, or a hydrogen atom,
$R^3$ is an alkyl group, a fluoroalkyl group, or an aryl group optionally having one or more substituent groups, and X is a fluoroalkyl group or a halogen atom,

(b) an alcohol, and
(c) a salt.

Item 29.

The composition according to Item 28, further comprising a polymerization inhibitor.

Item 30.

The composition according to Item 28 or 29, further comprising water.

Item 31.

The composition according to any one of claims 28 to 30, wherein the alcohol (b) is less volatile than in a composition consisting of the compound (a) and the alcohol (b).

Advantageous Effects of Invention

According to the present disclosure, a method for purifying an acrylic ester compound etc. is provided.

Description of Embodiments

The above summary of the present disclosure is not intended to describe each disclosed embodiment or every implementation of the present disclosure.
The description of the present disclosure that follows more specifically exemplifies illustrative embodiments.
In several places throughout the present disclosure, guidance is provided through lists of examples, and these examples can be used in various combinations.
In each instance, the described list serves only as a representative group, and should not be interpreted as an exclusive list.
All of the publications, patents, and patent applications cited herein are incorporated herein by reference in

their entirety.

Terms

The symbols and abbreviations in this specification can be understood to have the meanings commonly used in the technical field of the present disclosure in the context of the present description, unless otherwise specified.
The term "comprising" as used herein is intended to include "consisting essentially of" and "consisting of."
The steps, treatments, or operations described in this specification can be performed at room temperature, unless otherwise specified.
In this specification, the room temperature can mean a temperature in the range of 10 to 40°C.
In this specification, the notation "$C_{n-m}$" (wherein n and m are each a number) means that the number of carbon atoms is n or more and m or less, as is usually understood by persons skilled in the art.
In this specification, examples of the "halogen" include a fluorine atom, a chlorine atom, a bromine atom, and an iodine atom.
In this specification, the "alkyl" can be a linear, branched, or cyclic alkyl group.
In this specification, examples of the "alkyl" include $C_{1-20}$ alkyl, $C_{1-12}$ alkyl, $C_{1-6}$ alkyl, $C_{1-4}$ alkyl, and $C_{1-3}$ alkyl group.
In this specification, examples of the "alkyl" include linear or branched alkyl groups, such as methyl, ethyl, propyl (n-propyl, isopropyl), butyl (n-butyl, isobutyl, sec-butyl, tert-butyl), pentyl, and hexyl.
In this specification, examples of the "alkyl" include cyclic alkyl or cycloalkyl groups (e.g., $C_{3-8}$ cycloalkyl groups), such as cyclopropyl, cyclobutyl, cyclopentyl, and cyclohexyl.
In this specification, the "fluoroalkyl" is an alkyl group in which at least one hydrogen atom is replaced with a fluorine atom.
In this specification, the number of fluorine atoms in the "fluoroalkyl" can be 1 or more (e.g., 1 to 3, 1 to 6, 1 to 12, or 1 to the maximum substitutable number).
In this specification, the "fluoroalkyl" can be, for example, $C_{1-20}$ fluoroalkyl, $C_{1-12}$ fluoroalkyl, $C_{1-6}$ fluoroalkyl, $C_{1-4}$ fluoroalkyl, or $C_{1-3}$ fluoroalkyl.
In this specification, the "fluoroalkyl" can be a linear or branched fluoroalkyl group.
In this specification, the "fluoroalkyl" can be a perfluoroalkoxy group or a non-perfluoroalkyl group.
In this specification, examples of the "fluoroalkyl" include fluoromethyl, difluoromethyl, trifluoromethyl, 2,2,2-trifluoroethyl, pentafluoroethyl, tetrafluoropropyl (e.g., $HCF_2CF_2CH_2$), hexafluoropropyl (e.g., $(CF_3)_2CH$-), nonafluorobutyl, octafluoropentyl (e.g., $HCF_2CF_2CF_2CF_2CH_2$-), and tridecafluorohexyl.
In this specification, the "aryl" can be, for example, monocyclic, bicyclic, tricyclic, or tetracyclic.
In this specification, the "aryl" can be, for example, $C_{6-18}$ aryl, $C_{6-16}$ aryl, $C_{6-14}$ aryl, or $C_{6-12}$ aryl.
In this specification, examples of the "aryl" include phenyl, 1-naphthyl, 2-naphthyl, 2-biphenyl, 3-biphenyl, 4-biphenyl, and 2-anthryl.
In this specification, examples of substituents in the "aryl" include halogen atoms, a cyano group, an amino group, alkoxy groups, and alkylthio groups. Two or more substituents may be identical to or different from each other.
In this specification, the "alkoxy" can be a group in which an oxygen atom is bound to an alkyl group (alkyl-O-).
In this specification, the "alkoxy" can be a linear or branched alkoxy group.
In this specification, examples of the "alkoxy" include linear or branched $C_{1-20}$ alkoxy groups, such as methoxy, ethoxy, propoxy (n-propoxy, isopropoxy), butoxy (n-butoxy, isobutoxy, sec-butoxy, and tert-butoxy), pentyloxy, and hexyloxy.
In this specification, the "alkylthio" can be a group in which a sulfur atom is bound to an alkyl group (alkyl-S-).
In this specification, the "alkylthio" can be a linear or branched alkylthio group.
In this specification, examples of the "alkylthio" include linear or branched $C_{1-20}$ alkylthio groups, such as methylthio, ethylthio, propylthio (n-propylthio and isopropylthio), butylthio (e.g., n-butylthio, isobutylthio, secbutylthio, and tert-butylthio), pentylthio, and hexylthio.
The number of substituents in the aryl can be, for example, 1 or more (e.g., 1 to 3, 1 to 5, or 1 to the maximum substitutable number).

Purification Method

The purification method according to one embodiment of the present disclosure is a method for purifying a compound represented by formula (1):

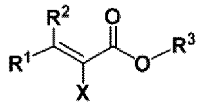
(1)

(wherein $R^1$ and $R^2$ are the same or different and represent an alkyl group, a fluoroalkyl group, an aryl group optionally having one or more substituent groups, a halogen atom, or a hydrogen atom,
$R^3$ is an alkyl group, a fluoroalkyl group, or an aryl group optionally having one or more substituent groups, and
X is a fluoroalkyl group or a halogen atom) (in this specification, this compound may be referred to as Compound (1)), the method comprising step A of distilling composition A containing

(a) the compound represented by formula (1),
(b) an alcohol, and
(c) a salt

to obtain a concentrate containing the compound of formula (1).

Step A

Composition A

Composition A is a target to be subjected to distillation and contains (a) a compound represented by formula (1), (b) an alcohol, and (c) a salt.
Preferably, composition A further contains water.
The lower limit of the amount of water in composition A is preferably 0.0001 mass%, more preferably 0.001 mass%, and even more preferably 0.01 mass%.
The upper limit of the amount of water in composition A is preferably 80 mass% and more preferably 70 mass%.
When composition A contains water, this can provide, for example, the following advantages.

(1) Composition A in the form of a solid or semi-solid (slurry) is inconvenient in that lumps tend to form easily, which makes uniform stirring difficult. Composition A containing water is preferable in that stirring and homogenization (liquefaction) become easy.
(2) Heating or cooling becomes easy due to the liquefaction in (1) above, which can save energy and time.
(3) The amount of adhesion to internal surfaces of reactors (e.g., reaction vessels) and pipes can be reduced due to the liquefaction in (1) above, and this can suitably reduce transfer loss and cleaning operations, and prevent problems caused by sticking.

Composition A may have any pH. For example, from the viewpoint of increasing the hydrolysis resistance of Compound (1), a neutral to acidic range is preferred. Specifically, the composition preferably has a pH of 7 or lower.
Composition A may contain a solvent (this solvent is not the water or alcohol (b) mentioned above).
The lower limit of the amount of the solvent is preferably 0.001 mass%, more preferably 0.01 mass%, and even more preferably 0.015 mass%. The upper limit of the amount of the solvent is preferably 80 mass%, more preferably 70 mass%, and even more preferably 60 mass%.
Composition A is preferably in the form of, for example, a suspension, a viscous fluid, a semi-solid, or a solid. Composition A is preferably a homogeneous product.
The salt (c), alcohol (b), and compound (1) are described below in this order.

Salt (c)

The salt (c) is preferably at least one salt selected from the group consisting of inorganic salts and organic salts.
The salt (c) can be preferably an inorganic salt.
Preferably, the quantitative ratio of the alcohol (b) to the salt (c) (alcohol (b)/salt (c)) is 0.01 mol/mol or more. More preferably, the salt (c) is solvated in the alcohol (b) that is used in a quantitative ratio of 0.01 mol/mol or more.
The quantitative ratio (alcohol (b)/salt (c)) is preferably 0.05 mol/mol or more, more preferably 0.1 mol/mol or more, and even more preferably 0.5 mol/mol or more.
The molar ratio (alcohol (b)/salt (c)) is usually 50 mol/mol or less, preferably 45 mol/mol or less, and more preferably 40 mol/mol or less.
The lower limit of the amount of the alcohol (b) per 100 parts by mass of the salt (c) is preferably 0.3 parts by

mass, more preferably 1.5 parts by mass, and even more preferably 3.0 parts by mass.

The upper limit of the amount of the alcohol (b) per 100 parts by mass of the salt (c) is preferably 1600 parts by mass, more preferably 1400 parts by mass, and even more preferably 1300 parts by mass.

The amount of the alcohol (b) relative to 100 parts by mass of the salt (c) is preferably in the range of 0.3 to 1600 parts by mass, more preferably in the range of 1.5 to 1400 parts by mass, and even more preferably in the range of 3.0 to 1300 parts by mass.

Preferably, the cation of the salt (c) is at least one member selected from the group consisting of metal cations, $NR^{4+}$ (wherein R in each occurrence is the same or different and represents H or an organic group, and any two of the Rs may be linked together to form an optionally substituted ring), optionally substituted imidazolium, optionally substituted pyridinium, and optionally substituted phosphonium.

The cation of the salt (c) can be, for example, a metal cation or an ammonium cation. Examples of the metal cation include monovalent metal cations (e.g., alkali metals such as Li and Na), divalent metal cations (e.g., alkali earth metals such as Ca), trivalent metal cations (e.g., Group 13 metals in the periodic table such as Al), and the like. Examples of the ammonium cation include quaternary ammonium cations. Specific examples include tetra-$C_{1-6}$ alkylammonium cations (e.g., tetramethylammonium cation, tetraethylammonium cation, tetrapropylammonium cation, and tetrabutylammonium cation).

The cation of the salt (c) can be preferably a metal cation.

Examples of the anion of the salt (c) include carbonate ions, hydrogen carbonate ions, carboxylate ions, sulfate ions, hydroxide ions, halogen ions (e.g., bromide ions, chloride ions, and iodine ions), nitrate ions, and the like.

The anion of the salt (c) can be preferably a halogen ion.

The inorganic salt used as the salt (c) is preferably a salt containing an alkaline earth metal or an alkali metal, or both.

The inorganic salt used as the salt (c) is preferably at least one member selected from the group consisting of $CaCl_2$, $MgCl_2$, LiI, LiCl, NaCl, and NaI.

The organic salt used as the salt (c) is preferably at least one member selected from calcium propionate and calcium salts of amino acids.

Preferably, the cation of the salt (c) is at least one member selected from the group consisting of monovalent metal cations, divalent metal cations, and trivalent metal cations.

Preferably, the anion of the salt (c) is at least one member selected from the group consisting of carbonate ions, hydrogen carbonate ions, carboxylate ions, sulfate ions, hydroxide ions, halogen ions, and nitrate ions.

Preferably, the salt (c) is at least one member selected from the group consisting of LiBr, LiI, $MgBr_2$, $MgI_2$, $ZnCl_2$, $ZnBr_2$, $AlCl_3$, $CaCl_2$, $MgCl_2$, LiCl, NaCl, and NaI.

Preferably, the salt (c) is at least one member selected from the group consisting of salts containing a carboxyl group, and salts having a molecular weight of 100 or more and a boiling point of 150°C or higher.

Preferably, the salt (c) is at least one member selected from the group consisting of calcium stearate, calcium tartrate, calcium propionate, and calcium salts of amino acids.

The lower limit of the concentration of the salt (c) in composition A is preferably 5%, more preferably 10%, even more preferably 15%, and still even more preferably 20%, relative to its saturation concentration.

The lower limit of the concentration of the salt (c) in composition A is preferably 25%, more preferably 30%, even more preferably 35%, still even more preferably 40%, particularly preferably 45%, and more particularly preferably 50%, relative to its saturation concentration.

The upper limit of the concentration of the salt (c) in composition A is preferably 99.0%, more preferably 99.3%, even more preferably 99.6%, and still even more preferably 100%, relative to its saturation concentration.

In this specification, the saturation concentration refers to a solubility limit concentration in water at the temperature at which distillation in step A is performed.

The concentration of the salt (c) in composition A is preferably 0.001 wt.% or more, more preferably 0.01 wt.% or more, and even more preferably 0.1 wt.% or more.

The concentration of the salt (c) in composition A is preferably 1 wt.% or more, more preferably 5 wt.% or more, and even more preferably 10 wt.% or more.

<u>Alcohol (b)</u>

Preferably, the alcohol (b) is a linear or branched alcohol having 1 to 5 carbon atoms.

Preferably, the alcohol (b) is an alcohol represented by formula:

$$R^4\text{-OH} \qquad (2)$$

(wherein $R^4$ is an alkyl group (preferably $C_{1-6}$ alkyl, more preferably $C_{1-4}$ alkyl, even more preferably $C_{1-2}$ alkyl, and

particularly preferably methyl) or a fluoroalkyl group (preferably $C_{1-6}$ fluoroalkyl, more preferably $C_{1-4}$ fluoroalkyl, even more preferably $C_{1-2}$ fluoroalkyl, and particularly preferably fluoromethyl, wherein the number of fluoro groups in the fluoroalkyl group can be any number in the range of 1 to the maximum substitutable number)).

Composition A may contain a polymerization inhibitor.

The lower limit of the amount of the polymerization inhibitor can be, for example, 0.0001 wt.%, 0.0005 wt.%, 0.001 wt.%, 0.005 wt.%, 0.01 wt.%, 0.05 wt.%, or 0.1 wt.%, based on the total amount of composition A.

The upper limit of the amount of the polymerization inhibitor can be, for example, 20 wt.%, 15 wt.%, 10 wt.%, 5 wt.%, 1 wt.%, 0.5 wt.%, 0.1 wt.%, or 0.05 wt.%, based on the total amount of composition A.

The amount of the polymerization inhibitor can be, for example, in the range of 0.0001 to 20 wt.%, 0.0005 to 5 wt.%, or 0.001 to 0.05 wt.%, based on the total amount of composition A.

Examples of the polymerization inhibitor include amine compounds, such as aliphatic primary amines, aliphatic secondary amines, aliphatic tertiary amines, alicyclic secondary amines, alicyclic tertiary amines, aromatic amines, heterocyclic amines, and polymer-supported amine compounds (polymeric amine compounds); ammonia; and terpene compounds; nitroso compounds (including tempo and dpph); and compounds having at least one atom selected from the group consisting of oxygen and sulfur.

Examples of aliphatic primary amines include methylamine, ethylamine, propylamine, butylamine, pentylamine, hexylamine, cyclohexylamine, and ethylenediamine.

Examples of aliphatic secondary amines include dimethylamine, diethylamine, dipropylamine, dibutylamine, dipentylamine, dihexylamine, and dicyclohexylamine.

Examples of aliphatic tertiary amines include trimethylamine, triethylamine, diisopropylethylamine, tri(n-butyl)amine, N,N,N',N'-tetramethylethylenediamine, and the like.

Examples of alicyclic secondary amines include piperidine, piperazine, pyrrolidine, morpholine, and the like.

Examples of alicyclic tertiary amines include N-methylpiperazine, N-methylpyrrolidine, 1,5-diazabicycio[4,3,0]nonan-5-ene, and 1,4-diazabicyclo[2,2,2]octane.

Examples of aromatic amines include aniline, methylaniline, dimethylaniline, N,N-dimethylaniline, haloaniline, and nitroaniline.

Examples of heterocyclic amines include pyridine, melamine, pyrimidine, piperazine, quinoline, and imidazole.

Examples of polymer-supported amine compounds (polymeric amine compounds) include polyethyleneimine, polyallylamine, and polyvinylpyridine.

Examples of terpene compounds include $\alpha$-pinene, camphene, $\alpha$-terpinene, D-limonene, $\gamma$-terpinene, p-cymene, and terpinolene.

Preferable examples of compounds having at least one atom selected from the group consisting of oxygen and sulfur include $C_{6-20}$ compounds having at least one 6-membered aromatic carbocyclic ring and at least one oxygen or sulfur atom directly attached to at least one ring-constituting carbon atom of the at least one 6-membered aromatic carbocyclic ring. Specific examples include hydroquinone, 4-methoxyphenol, 2,5-di-tert-butylhydroquinone, methylhydroquinone, tert-butylhydroquinone (TBH), p-benzoquinone, methyl-p-benzoquinone, tert-butyl-p-benzoquinone, 2,5-diphenyl-p-benzoquinone, 2,6-di-tert-butyl-4-methylphenol (BHT), phenothiazine, 4-tert-butylcatechol, 6-tert-butyl-2,4-xylenol, catechol, 2,6-di-tert-butylphenol, TEMPO, and DPPH.

Composition A can be prepared by mixing the compound (a), alcohol (b), and salt (c), optionally with water and other components as desired. The mixing can be performed by a known method (e.g., mixing using a mixer).

In the mixing, the addition of the compound (a), alcohol (b), salt (c), water, and other components can be performed in any order.

Specific examples of the addition include the following embodiments:

embodiment 1 in which the compound (a) and the alcohol (b) are added to the salt (c) (e.g., calcium chloride);
embodiment 2 in which an aqueous solution of the salt (c) (e.g., calcium chloride) is added to the compound (a) and the alcohol (b) ;
embodiment 3 in which water is added to the compound (a), the alcohol (b) and the salt (c) (e.g., calcium chloride);
embodiment 4 in which the salt (c) (e.g., calcium chloride) is added to the compound (a), the alcohol (b), and water;
embodiment 5 in which the salt (c) (e.g., calcium chloride) is added to the compound (a) and the alcohol (b); and
embodiment 6 in which the compound (a) and the alcohol (b) (e.g., a mixed solution of the compound (a) and the alcohol (b)) are added to the salt (c) (e.g., calcium chloride).

The amount of water as desired (this includes water in the aqueous solution) is preferably in the range of 0.001 to 80 mass%, more preferably in the range of 0.01 to 70 mass%, and even more preferably in the range of 0.1 to 65 mass%.

After the adjustment, composition A can be suitably allowed to stand until the composition is subjected to the distillation explained next. This can improve the recovery rate of the compound (a). The temperature at which composition A is allowed to stand can be, for example, room temperature. The time during which compound A is allowed to stand

can be, for example, 0.01 to 3 days.

Distillation

The distillation method is not limited. Examples include the direct distillation method, the thin film method, the continuous distillation method, the pressurized distillation method, the vacuum distillation method, the atmospheric distillation method, and pressure swing.

Preferably, composition A is directly distilled.

In the present disclosure, distillation broadly means separation based on the difference in boiling point. Just to note, the boiling point is not to be interpreted as limited to the normal boiling point etc., and may vary depending on various conditions, such as pressure. In other words, the boiling point can be changed by setting conditions as appropriate for the purpose of the technology of the present disclosure.

Examples of the distillation device that can be used in the present disclosure include distillation columns, distillers, conical dryers (vacuum rotary dryers), mixing dryers, and box dryers.

The distillation device is preferably configured to easily achieve a uniform state of the contents (for example, eliminate lumps of the contents).

Specifically, preferable examples include conical dryers having internal protrusions, devices having ribbon blades, and the like.

When the contents are in the form of a liquid, the distillation device is preferably a device capable of performing sufficient stirring. When the stirring is sufficient, the condensation effect is enhanced.

When composition A contains a polymerization inhibitor, nitrogen, air, or oxygen may be introduced into the reaction system. This introduction can be performed, for example, by bubbling.

The pressure conditions for the distillation can be, for example, 0 kPa or higher, preferably 0.01 kPa or higher, and more preferably 0.05 kPa or higher.

The pressure conditions for the distillation can be preferably 700 kPa or less, more preferably 650 kPa or less, and even more preferably 600 kPa or less.

Further, the pressure conditions for the distillation can be preferably 500 kPa or less, more preferably 400 kPa or less, even more preferably 300 kPa or less, still even more preferably 200 kPa or less, and particularly preferably 100 kPa or less.

The pressure conditions for the distillation can be preferably in the range of 0 to 700 kPa, more preferably in the range of 0.01 to 650 kPa, and even more preferably in the range of 0.05 to 600 kPa.

The conditions of the distillation can be preferably in the range of 0 to 100 kPa.

The temperature conditions for the distillation can be preferably -10°C or higher, more preferably -5°C or higher, even more preferably 1°C or higher, still even more preferably 5°C or more, and particularly preferably 10°C or more.

The temperature conditions for the distillation can be preferably 150°C or less, more preferably 145°C or less, and even more preferably 140°C or less.

Further, the temperature conditions for the distillation can be preferably 130°C or less, more preferably 120°C or less, even more preferably 110°C or less, and still even more preferably 100°C or less.

The temperature conditions for the distillation can be preferably in the range of -10 to 150°C, more preferably in the range of -5 to 145°C, and even more preferably from -1 to 140°C.

The temperature conditions for the distillation can be preferably be in the range of 10 to 100°C.

Preferably, $R^1$ is a hydrogen atom, an alkyl group, or a fluoroalkyl group.

$R^2$ is preferably a hydrogen atom, an alkyl group, or a fluoroalkyl group.

$R^3$ is preferably an alkyl group.

$R^3$ is preferably a $C_{1-4}$ alkyl group.

X is preferably a fluorine atom or a chlorine atom.

The purification method according to a preferred embodiment of the present disclosure further comprises step B of washing the concentrate obtained in step A with water or water containing an alkali metal compound or an alkaline earth metal compound, or containing both.

The purification method according to a preferred embodiment of the present disclosure further comprises step B of washing the concentrate obtained in step A with water or water containing at least one member selected from the group consisting of lithium chloride, lithium bromide, magnesium bromide, magnesium iodide, magnesium sulfate, lithium iodide, sodium iodide, magnesium chloride, and calcium chloride.

The purification method according to a more preferred embodiment of the present disclosure further comprises step B of washing the concentrate obtained in step A with water containing calcium chloride.

The expression "water containing (a substance)" used herein means a state in which the substance is present in water. The substance may have various forms in water, which depend on its inherent properties and the presence of other substances in the water. For example, the substance may be dissolved in water or dissociated into ions, or may

exist in the form of a solid.

The conditions for the washing can be appropriately determined in light of common technical knowledge.

The purification method according to the present disclosure preferably further comprises step C of distilling the residue after obtaining the concentrate in step A so as to recover the alcohol and the salt. The conditions for the distillation can be appropriately determined in light of common technical knowledge.

Composition

The composition according to the present disclosure comprises a composition comprising

(a) a compound represented by formula (1):

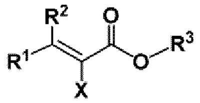

(1)

wherein

($R^1$ and $R^2$ are the same or different and represent an alkyl group, a fluoroalkyl group, an aryl group optionally having one or more substituent groups, a halogen atom, or a hydrogen atom,
$R^3$ is an alkyl group, a fluoroalkyl group, or an aryl group optionally having one or more substituent groups, and X is a fluoroalkyl group or a halogen atom),

(b) an alcohol, and
(c) a salt.

The composition can be composition A described above. The composition can be understood with reference to the above descriptions about composition A.

The salt (c) in the composition according to a preferred embodiment is preferably at least one member selected from the group consisting of $NH_4F$, $NH_4Br$, $N(C_nH_{2n+1})_4F$ (wherein n is 1 to 6), $(NH_4)_2NO_3$, $(NH_4)_2S_2O_3$, $NH_4HCO_3$, $NaHCO_3$, $NaHSO_4$, $Na_2SO_3$, $Na_2CO_3$, $Na_2S_2O_3$, $Na_2SO_4$, NaOH, NaCl, NaF, NaI, NaBr, $LiHCO_3$, $Li_2SO_3$, $Li_2CO_3$, $Li_2S_2O_3$, $Li_2SO_4$, LiOH, LiCl, LiI, LiBr, $KHCO_3$, $KHSO_4$, $K_2SO_3$, $K_2CO_3$, $K_2S_2O_3$, $K_2SO_4$, KOH, KCl, KF, KI, KBr, $MgSO_3$, $MgCO_3$, $MgS_2O_3$, $MgSO_4$, $Mg(OH)_2$, $MgCl_2$, $MgI_2$, $MgBr_2$, MgO, $Mg(CH_3COO)_2$, $Ca(HCO_3)_2$, $CaSO_3$, $CaCO_3$, $CaSO_4$, $Ca(OH)_2$, $CaCl_2$, $CaI_2$, $CaBr_2$, CaO, $Ca(NO_3)_2$, $Ca_3(PO_4)_2$, $Ca(CH_3COO)_2$, $Al_2(CO_3)_3$, $Al_2(SO_4)_3$, $Al(OH)_3$, $AlCl_3$, $AlBr_3$, $Al_2O_3$, $Al(NO_3)_3$, $AlPO_4$, $ZnSO_4$, $Zn(OH)_2$, $ZnCl_2$, $ZnBr_2$, $ZnI_2$, ZnO, $Zn(NO_3)_2$, $Zn_3(PO_4)_2$, $Zn(CH_3COO)_2$, calcium stearate, calcium tartrate, calcium propionate, calcium benzoate, calcium lactate, and calcium salts of amino acids;

more preferably at least one member selected from the group consisting of $NH_4F$, $NH_4Br$, $N(C_nH_{2n+1})_4F$ (wherein n is 1 to 6), $(NH_4)_2NO_3$, $Na_2SO_3$, $Na_2CO_3$, $Na_2SO_4$, NaI, $Li_2SO_3$, $Li_2CO_3$, $Li_2SO_4$, LiCl, LiI, LiBr, $K_2SO_3$, $K_2CO_3$, $K_2SO_4$, KCl, KF, KI, KBr, $MgSO_3$, $MgCO_3$, $MgSO_4$, $Mg(OH)_2$, $MgCl_2$, $MgI_2$, $MgBr_2$, MgO, Mg $(CH_3COO)_2$, $CaSO_3$, $CaCO_3$, $CaSO_4$, $Ca(OH)_2$, $CaCl_2$, $CaI_2$, $CaBr_2$, CaO, $Ca(NO_3)_2$, $Ca_3(PO_4)_2$, $Ca(CH_3COO)_2$, $Al_2(CO_3)_3$, $Al_2(SO_4)_3$, $Al(OH)_3$, $AlCl_3$, $AlBr_3$, $Al_2O_3$, $Al(NO_3)_3$, $AlPO_4$, $ZnSO_4$, $Zn(OH)_2$, $ZnCl_2$, $ZnBr_2$, ZnO, $Zn(NO_3)_2$, $Zn_3(PO_4)_2$, $Zn(CH_3COO)_2$, calcium stearate, calcium tartrate, calcium propionate, and calcium salts of amino acids; and
even more preferably at least one member selected from the group consisting of $N(C_nH_{2n+1})_4F$ (wherein n is 1 to 6), $Na_2SO_4$, NaI, $Li_2SO_4$, LiCl, LiI, LiBr, $K_2SO_4$, KCl, KI, KBr, $MgCO_3$, $MgSO_4$, $Mg(OH)_2$, $MgCl_2$, $MgI_2$, $MgBr_2$, MgO, $Mg(CH_3COO)_2$, $CaCO_3$, $CaSO_4$, $Ca(OH)_2$, $CaCl_2$, $CaI_2$, $CaBr_2$, CaO, $Ca(CH_3COO)_2$, $Al_2(SO_4)_3$, $Al(OH)_3$, $AlCl_3$, $AlBr_3$, $Al_2O_3$, $AlPO_4$, $ZnSO_4$, $Zn(OH)_2$, $ZnCl_2$, $ZnBr_2$, ZnO, $Zn(CH_3COO)_2$, calcium stearate, calcium tartrate, calcium propionate, and calcium salts of amino acids.

The amount of the salt is preferably 0.001 to 99.99 mass%, more preferably 0.01 to 99 mass%, and even more preferably 0.1 to 95 mass%, based on the total amount of the composition.

In the composition according to a preferred embodiment, the polymerization inhibitor is preferably, for example, an amine compound, a nitroso compound, or an OH-containing compound.

Preferable examples of the OH-containing compound include hydroquinone, 4-methoxyphenol, 2,5-di-tert-butylhydroquinone, methylhydroquinone, tert-butylhydroquinone (TBH), p-benzoquinone, methyl-p-benzoquinone, tert-butyl-p-benzoquinone, 2,5-diphenyl-p-benzoquinone, 2,6-di-tert-butyl-4-methylphenol (BHT), 4-tert-butyl catechol, 6-tert-butyl-2,4-xylenol, catechol, and 2,6-di-tert butylphenol.

The lower limit of the amount of the polymerization inhibitor can be, for example, 0.0001 wt.%, 0.0005 wt.%, 0.001 wt.%, 0.005 wt.%, 0.01 wt.%, 0.05 wt.%, or 0.1 wt.%, based on the total amount of the composition, based on the total amount of the composition.

The upper limit of the amount of the polymerization inhibitor can be, for example, 20 wt.%, 15 wt.%, 10 wt.%, 5 wt.%, 1 wt.%, 0.5 wt.%, 0.1 wt.%, or 0.05 wt.%, based on the total amount of the composition.

The amount of the polymerization inhibitor can be, for example, in the range of 0.0001 to 20 wt.%, in the range of 0.0005 to 5 wt.%, or in the range of 0.001 to 0.05 wt.%, based on the total amount of the composition.

The volatilization of the alcohol (b) from the above composition is suppressed as compared with that in the corresponding composition consisting of the compound (a) and the alcohol (b) (or the same composition as the above composition, except that the composition does not contain the salt (c)).

The volatilization can be measured, for example, by subjecting the composition to distillation under reduced pressure (10 kPa, 24°C) and measuring the amount of the alcohol (b) in the distillate. The volatilization can be confirmed by measuring the amount of the alcohol (b) in the distillate.

The volatilization inhibition rate (%) can be used as an indicator for the degree of inhibition of volatilization. The volatilization inhibition rate (%) (which may be simply referred to below as the "inhibition rate") is preferably in the range of 10% to 100%, more preferably 20% to 100%, and even more preferably 30% to 100%.

The inhibition rate can be expressed by the following formula:

$$\text{Inhibition rate (\%)} = (X1-X2)/X1 \times 100$$

In the formula, X1 represents the amount of alcohol (b) in the distillate obtained by distillation of the composition consisting of the compound (a) and alcohol (b), and X2 is the amount of the alcohol (b) in the distillate obtained by distillation of composition (A).

Thus, the composition can be stably stored.

The above describes embodiments of the present disclosure. However, various modifications of the embodiments and details can be made without departing from the spirit and scope of the claims.

## Examples

The present invention is described in more detail below with reference to Examples. However, the present invention is not limited to these Examples.

### Example 1

100 g of $CaCl_2$ was added to 100 g of a mixed solution of 20 wt.% of 2-fluoroacrylic acid methyl ester and 80 wt.% of MeOH (methanol). The obtained solid was allowed to stand for 48 hours and then subjected to distillation under reduced pressure (conditions: 10 kPa, 24°C) to obtain a mixture of 70 wt.% of 2-fluoroacrylic acid methyl ester and 30 wt.% of MeOH as a distillate. In this case, the recovery of the 2-fluoroacrylic acid methyl ester was 99%. The inhibition rate was 89%.

### Example 2

100 g of $CaCl_2$ was added to 100 g of a mixed solution of 20 wt.% of 2-fluoroacrylic acid methyl ester and 80 wt.% of MeOH. The obtained solid was distilled under reduced pressure (10 kPa, 24°C) to obtain a mixture of 67 wt.% of the 2-fluoroacrylic acid methyl ester and 33 wt.% of MeOH as a distillate. In this case, the recovery of the 2-fluoroacrylic acid methyl ester was 94%. The inhibition rate was 88%.

### Example 3

100 g of $CaCl_2$ and 110 g of water were added to 100 g of a mixed solution of 15 wt.% of 2-fluoroacrylic acid methyl ester and 85 wt.% of MeOH. The resulting mixture had a pH of 5. The obtained liquid was distilled under reduced pressure (5 kPa, 24°C) to obtain a mixture of 59 wt.% of the 2-fluoroacrylic acid methyl ester, 39 wt.% of MeOH, and 2 wt.% of water as a distillate. In this case, the recovery of the 2-fluoroacrylic acid methyl ester was 97%. The inhibition rate was 89%.

Example 4

After the distillation of Example 3, distillation was performed under reduced pressure while gradually increasing the temperature (5 kPa, 30 to 100°C) to obtain MeOH as a distillate. The recovery rate of MeOH was 99%. The recovery rate of $CaCl_2$ was 100%.

Example 5

110 g of a 47 wt.% aqueous $CaCl_2$ solution was added to 100 g of a mixed solution of 59 wt.% of 2-fluoroacrylic acid methyl ester, 39 wt.% of MeOH, and 2 wt.% of water, and the resulting mixture was stirred. The two phases were separated to obtain a mixture of 98 wt.% of the 2-fluoroacrylic acid methyl ester and MeOH of 2 wt%. The recovery rate of the 2-fluoroacrylic acid methyl ester was 91%.

As impurities, the following compounds were individually confirmed by gas chromatography (<1 GC area %):

(1) an ester compound (methyl acetate);
(2) a compound obtained by acetalizing an ester compound with an alcohol (a compound obtained by acetalizing methyl acetate with alcohol (MeOH));
(3) a compound obtained by hydrolyzing an ester compound with water (acetic acid, 2-fluoroacrylic acid, and/or salts thereof);
(4) a compound obtained by adding an alcohol to an acrylic acid compound (a compound obtained by adding an alcohol (MeOH) to 2-fluoroacrylic acid methyl ester or 2-fluoroacrylic acid); and
(5) a compound obtained by adding water to an acrylic acid compound (a compound obtained by adding water to 2-fluoroacrylic acid methyl ester or 2-fluoroacrylic acid).

Example 6

100 g of $CaCl_2$, 110 g of water, and 0.015 g of hydroquinone were added to 100 g of a mixed solution of 15 wt.% of 2-fluoroacrylic acid methyl ester and 85 wt.% of MeOH. The obtained liquid was subjected to distillation under reduced pressure (5 kPa, 24°C) to obtain a mixture of 59 wt.% of the 2-fluoroacrylic acid methyl ester, 40 wt.% of MeOH, and 1 wt.% of water as a distillate. In this case, the recovery rate of the 2-fluoroacrylic acid methyl ester was 98%. The inhibition rate was 88%.

Example 7

100 g of $CaCl_2$, 110 g of water, and 0.3 g of BHT were added to 100 g of a mixed solution of 15 wt.% of 2-fluoroacrylic acid methyl ester and 85 wt.% of MeOH. The obtained liquid was subjected to distillation under reduced pressure (5 kPa, 24°C) to obtain a mixture of 49 wt.% of the 2-fluoroacrylic acid methyl ester, 49 wt.% of MeOH, and 2 wt.% of water as a distillate. The recovery rate of the 2-fluoroacrylic acid methyl ester was 98%. The inhibition rate was 83%.

Example 8

100 g of $CaCl_2$, 110 g of water, and 0.015 g of 4-methoxyphenol were added to 100 g of a mixed solution of 15 wt.% of 2-fluoroacrylic acid methyl ester and 85 wt.% of MeOH. The obtained liquid was subjected to distillation (using a Vigreux column) under reduced pressure (5 kPa, 24°C) to obtain a mixture of 58.5 wt.% of the 2-fluoroacrylic acid methyl ester, 40 wt.% of MeOH, and 1.5 wt.% of water as a distillate. In this case, the recovery of the 2-fluoroacrylic acid methyl ester was 98%. The inhibition rate was 88%.

Example 9

100 g of $CaCl_2$, 110 g of water, and 0.015 g of TBH were added to 100 g of a mixed solution of 15 wt.% of 2-fluoroacrylic acid methyl ester and 85 wt.% of MeOH. The obtained liquid was subjected to distillation (using a Vigreux column) under reduced pressure (5 kPa, 24°C) to obtain a mixture of 54 wt.% of the 2-fluoroacrylic acid methyl ester, 44.5 wt.% of MeOH, and 1.55 wt.% of water as a distillate. The recovery of the 2-fluoroacrylic acid methyl ester was 96%. The inhibition rate was 86%.

Example 10

Hydrochloric acid was added to 100 g of a mixed solution containing 15 g of 2-fluoroacrylic acid methyl ester, 85 g of MeOH, and 0.015 g of 4-methoxyphenol to adjust the pH to 1. 100 g of $CaCl_2$ and 110 g of water were added to the resulting solution. The obtained liquid was subjected to distillation under reduced pressure (5 kPa, 24°C) to obtain a mixture of 58.5 wt.% of the 2-fluoroacrylic acid methyl ester, 40 wt.% of MeOH, and 1.5 wt.% of water as a distillate. The recovery rate of the 2-fluoroacrylic acid methyl ester was 98%. The inhibition rate was 88%.

Example 11

60 g of LiCl was added to 100 g of a mixed solution of 15 wt.% of 2-fluoroacrylic acid methyl ester and 85 wt.% of MeOH. The obtained liquid was subjected to distillation under reduced pressure (5 kPa, 25°C) to obtain a mixture of 39 wt.% of the 2-fluoroacrylic acid methyl ester and 61 wt.% of MeOH as a distillate. In this case, the recovery rate of the 2-fluoroacrylic acid methyl ester was 100%. The inhibition rate was 72%.

Example 12

204 g of NaI was added to 100 g of a mixed solution of 15 wt.% of 2-fluoroacrylic acid methyl ester and 85 wt.% of MeOH. The obtained liquid was subjected to distillation under reduced pressure (5 kPa, 25°C) to obtain a mixture of 20 wt.% of the 2-fluoroacrylic acid methyl ester and 80 wt.% of MeOH as a distillate. The recovery rate of the 2-fluoroacrylic acid methyl ester was 100%. The inhibition rate was 29%.

Example 13

103 g of $Na_2SO_4$ was added to 100 g of a mixed solution of 15 wt.% of 2-fluoroacrylic acid methyl ester and 85 wt.% of MeOH. The obtained liquid was subjected to distillation under reduced pressure (5 kPa, 25°C) to obtain a mixture of 44 wt.% of the 2-fluoroacrylic acid methyl ester and 56 wt.% of MeOH. In this case, the recovery rate of the 2-fluoroacrylic acid methyl ester was 100%. The inhibition rate was 78%.

Example 14

142 g of $MgCl_2$ was added to 100 g of a mixed solution of 15 wt.% of 2-fluoroacrylic acid methyl ester and 85 wt.% of MeOH. The obtained liquid was subjected to distillation under reduced pressure (5 kPa, 25°C) to obtain a mixture of 43 wt.% of the 2-fluoroacrylic acid methyl ester and 57 wt.% of MeOH as a distillate. The recovery rate of the 2-fluoroacrylic acid methyl ester was 100%. The inhibition rate was 76%.

## Claims

**1.** A method for purifying a compound represented by formula (1):

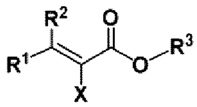 (1)

wherein

$R^1$ and $R^2$ are the same or different and represent an alkyl group, a fluoroalkyl group, an aryl group optionally having one or more substituent groups, a halogen atom, or a hydrogen atom,
$R^3$ is an alkyl group, a fluoroalkyl group, or an aryl group optionally having one or more substituent groups, and
X is a fluoroalkyl group or a halogen atom,
the method comprising
step A of distilling
composition A containing

(a) the compound represented by formula (1),
(b) an alcohol, and

(c) a salt

to obtain a concentrate containing the compound of formula (1).

**2.** The method according to claim 1, wherein composition A further contains water.

**3.** The method according to claim 1 or 2, wherein composition A is homogeneous.

**4.** The method according to any one of claims 1 to 3, wherein the salt (c) is at least one member selected from the group consisting of inorganic salts and organic salts.

**5.** The method according to any one of claims 1 to 4, wherein the quantitative amount ratio of the alcohol (b) to the salt (c) (alcohol (b)/salt (c)) is 0.01 mol/mol or more.

**6.** The method according to any one of claims 1 to 5, wherein the salt (c) is solvated in the alcohol (b) that is used in a quantitative ratio of 0.5 mol/mol or more.

**7.** The method according to any one of claims 1 to 6,

wherein the salt (c) comprises at least one cation selected from the group consisting of
metal cations,
$NR_4^+$ wherein R in each occurrence is the same or different and represents H or an organic group, or any two of the Rs may be linked together to form an optionally substituted ring,
optionally substituted imidazolium,
optionally substituted pyridinium, and
optionally substituted phosphonium.

**8.** The method according to any one of claims 1 to 7, wherein the salt (c) comprises at least one cation selected from the group consisting of monovalent metal cations, divalent metal cations, and trivalent metal cations.

**9.** The method according to any one of claims 1 to 8, wherein the salt (c) comprises at least one anion selected from the group consisting of carbonate ions, hydrogen carbonate ions, carboxylate ions, sulfate ions, hydroxide ions, halogen ions, and nitrate ions.

**10.** The method according to any one of claims 1 to 9, wherein the salt (c) is at least one member selected from the group consisting of LiBr, LiI, $MgBr_2$, $MgI_2$, $ZnCl_2$, $ZnBr_2$, $AlCl_3$, $CaCl_2$, $MgCl_2$, LiCl, NaCl, and NaI.

**11.** The method according to any one of claims 1 to 9, wherein the salt (c) is at least one member selected from the group consisting of calcium stearate, calcium tartrate, calcium propionate, and calcium salts of amino acids.

**12.** The method according to any one of claims 1 to 11, wherein composition A contains the salt (c) at a concentration of 50% or more, relative to its saturation concentration.

**13.** The method according to any one of claims 1 to 12, wherein the concentration of the salt (c) in composition A is 10 wt.% or more.

**14.** The method according to any one of claims 1 to 13, wherein the alcohol (b) is a linear or branched alcohol having 1 to 5 carbon atoms.

**15.** The method according to any one of claims 1 to 14, wherein the alcohol (b) is represented by formula:

$$R^4\text{-OH} \qquad (2)$$

wherein $R^4$ is an alkyl group or a fluoroalkyl group.

**16.** The method according to any one of claims 1 to 15, wherein composition A is directly distilled.

**17.** The method according to any one of claims 1 to 16, wherein the distillation is performed at a pressure of 0 to 100 kPa.

**18.** The method according to any one of claims 1 to 17, wherein the distillation is performed at a temperature of 10 to 100°C.

**19.** The method according to any one of claims 1 to 18, wherein $R^1$ is a hydrogen atom, an alkyl group, or a fluoroalkyl group.

**20.** The method according to any one of claims 1 to 19, wherein $R^2$ is a hydrogen atom, an alkyl group, or a fluoroalkyl group.

**21.** The method according to any one of claims 1 to 20, wherein $R^3$ is an alkyl group.

**22.** The method according to any one of claims 1 to 21, wherein $R^3$ is a $C_{1-4}$ alkyl group.

**23.** The method according to any one of claims 1 to 22, wherein X is a fluorine atom or a chlorine atom.

**24.** The method according to any one of claims 1 to 23, further comprising step B of washing the concentrate obtained in step A with water or water containing an alkali metal compound or an alkaline earth metal compound, or containing both.

**25.** The method according to any one of claims 1 to 23, further comprising step B of washing the concentrate obtained in step A with water or water containing at least one member selected from the group consisting of lithium chloride, lithium bromide, magnesium bromide, magnesium iodide, magnesium sulfate, lithium iodide, sodium iodide, magnesium chloride, and calcium chloride.

**26.** The method according to any one of claims 1 to 23, further comprising step B of washing the concentrate obtained in step A with water containing calcium chloride.

**27.** The method according to any one of claims 1 to 26, further comprising step C of distilling the residue after obtaining the concentrate in step A so as to recover the alcohol and the salt.

**28.** A composition comprising

(a) a compound represented by formula (1):

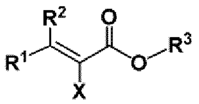 (1)

wherein

$R^1$ and $R^2$ are the same or different and are an alkyl group, a fluoroalkyl group, an aryl group optionally having one or more substituent groups, a halogen atom, or a hydrogen atom,
$R^3$ is an alkyl group, a fluoroalkyl group, or an aryl group optionally having one or more substituent groups, and
X is a fluoroalkyl group or a halogen atom,

(b) an alcohol, and
(c) a salt.

**29.** The composition according to claim 28, further comprising a polymerization inhibitor.

**30.** The composition according to claim 28 or 29, further comprising water.

**31.** The composition according to any one of claims 28 to 30, wherein the alcohol (b) is less volatile than in a composition consisting of the compound (a) and the alcohol (b).

## INTERNATIONAL SEARCH REPORT

International application No.
**PCT/JP2021/030466**

**A. CLASSIFICATION OF SUBJECT MATTER**

***C07C 67/54***(2006.01)i; ***C07C 69/653***(2006.01)i
FI: C07C67/54; C07C69/653

According to International Patent Classification (IPC) or to both national classification and IPC

**B. FIELDS SEARCHED**

Minimum documentation searched (classification system followed by classification symbols)

C07C67/54; C07C69/653

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2017-36231 A (DAIKIN IND LTD) 16 February 2017 (2017-02-16) claims, production example 1, paragraphs [0035], [0018] | 1, 3-7, 12-23, 27-29, 31<br>24-26 |
| Y | WO 2001/030739 A1 (DAICEL CHEMICAL INDUSTRIES, LTD.) 03 May 2001 (2001-05-03) claims, page 14 | 24-26 |
| Y | JP 2003-231665 A (MITSUBISHI CHEMICALS CORP) 19 August 2003 (2003-08-19) claims, etc. | 24-26 |
| A | WO 2005/102984 A1 (DAIKIN INDUSTRIES, LTD.) 03 November 2005 (2005-11-03) claims, examples, etc. | 1-31 |
| A | JP 2014-214147 A (DAIKIN IND LTD) 17 November 2014 (2014-11-17) claims, examples, etc. | 1-31 |
| A | WO 2019/193925 A1 (OSAKA ORGANIC CHEMICAL INDUSTRY LTD.) 10 October 2019 (2019-10-10) claims, examples, etc. | 1-31 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 October 2021** | **19 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2021/030466

C. DOCUMENTS CONSIDERED TO BE RELEVANT

| Category* | Citation of document, with indication, where appropriate, of the relevant passages | Relevant to claim No. |
|---|---|---|
| A | JP 2010-37248 A (NIPPON SHOKUBAI CO LTD) 18 February 2010 (2010-02-18) claims, examples, etc. | 1-31 |

Form PCT/ISA/210 (second sheet) (January 2015)

**INTERNATIONAL SEARCH REPORT**
**Information on patent family members**

International application No.
**PCT/JP2021/030466**

| Patent document cited in search report | Publication date (day/month/year) | Patent family member(s) | Publication date (day/month/year) |
|---|---|---|---|
| JP 2017-36231 A | 16 February 2017 | (Family: none) | |
| WO 2001/030739 A1 | 03 May 2001 | JP 5220253 B2 | |
| JP 2003-231665 A | 19 August 2003 | WO 2003/045894 A1 | |
| | | CN 1550489 A | |
| | | CN 1617847 A | |
| WO 2005/102984 A1 | 03 November 2005 | US 2007/0232766 A1 | |
| | | EP 1757578 A1 | |
| | | CN 1946669 A | |
| JP 2014-214147 A | 17 November 2014 | (Family: none) | |
| WO 2019/193925 A1 | 10 October 2019 | (Family: none) | |
| JP 2010-37248 A | 18 February 2010 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S62132925 A